## Europäisches Patentamt

(19) European Patent Office

Office européen des brevets

(11) **EP 0 652 197 B1**

(12) **FASCICULE DE BREVET EUROPEEN**

(45) Date de publication et mention
de la délivrance du brevet:
**22.04.1998  Bulletin 1998/17**

(51) Int. Cl.$^6$: **C07C 41/01**, C07C 41/06, C07C 43/04

(21) Numéro de dépôt: **94402268.0**

(22) Date de dépôt: **10.10.1994**

(54) **Procédé de production d'au moins un alkyl tertiobutyl éther à partir du gaz naturel**

Verfahren zur Herstellung von mindestens einem Alkyl Tertiär-Butylether aus Erdgas

Production of at least one alkyl t-butyl ether from natural gas

(84) Etats contractants désignés:
**BE DE ES FR GB IT NL SE**

(30) Priorité:  **15.10.1993 FR 9312404**

(43) Date de publication de la demande:
**10.05.1995  Bulletin 1995/19**

(73) Titulaires:
- **INSTITUT FRANCAIS DU PETROLE**
  **92502 Rueil-Malmaison (FR)**
- **ELF AQUITAINE PRODUCTION**
  **92400 Courbevoie (FR)**

(72) Inventeurs:
- **Cameron, Charles**
  **75005 Paris (FR)**
- **Chaumette, Patrick**
  **F-78380 Bougival (FR)**
- **Dang Vu, Quang**
  **F-92200 Neuilly sur Seine (FR)**
- **Bousquet, Jacques**
  **F-69540 Irigny (FR)**
- **Tournier-Lasserve, Jacques**
  **F-64000 Pau (FR)**
- **Desgrandchamps, Guy**
  **F-64140 Billere (FR)**

(56) Documents cités:
EP-A- 0 336 823          GB-A- 2 080 297
GB-A- 2 123 411          GB-A- 2 227 249
US-A- 4 731 490

- HYDROCARBON PROCESSING, vol.69, no.12, Décembre 1990, HOUSTON US pages 34B - 34D J FOREMAN 'ADDING ANOTHER STEP TO STEAM REFORMING SAVES FUEL AND BOOSTS OUTPUT'

Il est rappelé que:  Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

**Description**

La présente invention concerne un procédé de production d'au moins un alkyl tertiobutyl éther au moins partiellement à partir du gaz naturel.

Le gaz naturel est une matière première fossile abondante, constituée essentiellement de méthane, dont les réserves prouvées actuellement, de l'ordre de $10^{14}$ m$^3$, représentent environ 50 années de consommation mondiale. Les gisements de gaz contiennent souvent des quantités importantes d'éthane, de propane, d'autres alcanes supérieurs (des hydrocarbures $C_3^+$, c'est-à-dire des hydrocarbures comportant un nombre d'atomes de carbone par molécule au moins égal à 3), ainsi que d'autres constituants tels que $H_2O$, $CO_2$, $N_2$, $H_2S$ et He. La majorité du propane et des autres alcanes supérieurs associés au gaz naturel sont liquéfiés et désignés sous le nom de GPL (gaz de pétrole liquéfié). Dans les gisements riches en hélium (généralement plus de 0,3% en volume), l'hélium est séparé à cause de sa haute valeur commerciale. On sépare également l'hydrogène sulfuré, à cause de son caractère corrosif, et l'eau, à cause de la formation d'hydrates qui nuisent au transport du gaz naturel. Le gaz naturel purifié obtenu aprés déshydratation et désulfuration, de préférence jusqu'à une teneur en composés soufrés inférieure à 10 ppm, est alors qualifié de gaz non condensé, et contient principalement du méthane (par exemple 55-99% en volume) ainsi que de l'éthane, généralement du propane et éventuellement de faibles quantités d'azote et/ou de dioxyde de carbone. Toutefois, par abus de langage, on appelle généralement gaz naturel indifféremment le gaz purifié, obtenu après déshydratation et désulfuration, ou le gaz non purifié, issu d'un gisement et n'ayant subi aucune purification préalable.

La plus grande partie du gaz naturel est utilisée pour le chauffage individuel et industriel. Cependant, certains procédés de transformation du gaz naturel en hydrocarbures supérieurs existent.

La transformation du gaz naturel en alkyl tertiobutyl éther(s), tels que par exemple respectivement le méthyl tertiobutyl éther (MTBE) ou l'éthyl tertiobutyl éther (ETBE), est un objectif hautement désirable. En effet, les alkyl tertiobutyl éthers sont actuellement produits par réaction de l'isobutène avec un alcool, tels que par exemple respectivement le méthanol ou l'éthanol, et si lesdits alcools sont aisément synthétisés à partir du gaz naturel via le gaz de synthèse (défini comme un mélange contenant du monoxyde de carbone, de l'hydrogène et éventuellement du dioxyde de carbone), il n'en est pas de même pour l'isobutène qui est habituellement extrait d'une coupe $C_4$ (hydrocarbures comprenant 4 atomes de carbone par molécule), disponible par exemple en sortie d'une unité de craquage catalytique de coupes pétrolières. Cette source d'oléfines paraît actuellement beaucoup trop limitée pour faire face à la demande croissante en alkyl tertiobutyl éther(s) tels que le MTBE ou l'ETBE. Dans ce contexte, il est intéressant de trouver une méthode permettant de produire des alkyl tertiobutyl éthers à partir du gaz naturel.

Un des objectifs du procédé selon la présente invention est de synthétiser au moins un alkyl tertiobutyl éther, de préférence respectivement le MTBE ou l'ETBE, à partir d'au moins un alcool et à partir d'isobutène, synthétisés au moins partiellement à partir du gaz naturel. Dans le procédé selon l'invention, il est ainsi proposé de réaliser la synthèse d'alcool, de préférence respectivement de méthanol ou d'éthanol, à partir de gaz de synthèse, ledit gaz de synthèse étant en partie préparé dans une zone de préréformage à la vapeur du gaz naturel, et de synthétiser l'isobutène au moyen d'un enchaînement de procédé comprenant entre autres la transformation directe du gaz naturel en éthylène, communément appelée couplage oxydant du méthane (C.O.M.), la dimérisation de l'éthylène en normal n-butène, l'isomérisation du n-butène en isobutène, et les unités de séparation associées. Le procédé d'obtention d'au moins un alkyl tertiobutyl éther à partir du gaz naturel est appelé procédé d'oxyétherification

Le brevet US-A-5 159 125 revendique un procédé de condensation aldolique du méthanol ou d'autres alcools avec une oléfine comprenant au moins deux atomes de carbone, afin de synthétiser un mélange d'alcools de poids moléculaires supérieurs riche en isobutanol. L'isobutanol peut ensuite être séparé et déshydraté en isobutène et ledit isobutène condensé avec le méthanol pour former du MTBE, ces procédés étant connus de l'homme du métier.

Un autre objectif du procédé selon l'invention est de valoriser les sous-produits provenant de l'oxydation non sélective du gaz naturel dans le réacteur C.O.M., c'est-à-dire le monoxyde de carbone et le dioxyde de carbone, ainsi que l'hydrogène coproduit, en tant que gaz de synthèse servant à alimenter au moins partiellement l'unité de synthèse d'au moins un alcool.

Pour produire de l'éthylène et d'autres hydrocarbures, il a déjà été proposé d'effectuer le couplage oxydant du méthane ou d'une coupe contenant majoritairement du méthane (C.O.M.) soit en mode séquentiel, soit en mode simultané.

La réaction du couplage oxydant en mode séquentiel consiste en l'oxydation du méthane par un agent réductible suivie de la réoxydation de cet agent, séparément, par l'oxygène de l'air. L'utilisation de nombreux oxydes de métaux, notamment Mn, Ce, Pr, Sn, In, Ge, Pb, Sb, Bi, Tb, comme agents réductibles pour cette réaction, a été mentionnée dans plusieurs brevets américains tels que, par exemple, US-A-4 499 323, US-A-4 523 049, US-A-4 547 611 et US-A-4 567 307.

La réaction du couplage oxydant en mode simultané (balayage d'un mélange de méthane et d'oxygène sur une masse de contact) peut s'écrire qualitativement :

$$CH_4 + O_2 \rightarrow C_2H_6 + C_2H_4 + \text{autres hydrocarbures} + CO + CO_2 + H_2 + H_2O$$

Plusieurs brevets mentionnent (voir, par exemple, les demandes de brevet européen EP-A2-210 383, EP-A1-189 079, EP-A1-206 044 et la demande de brevet PCT WO 86 07351) l'utilisation d'oxydes de terres rares, d'oxydes alcalins et alcalino-terreux, et d'oxydes de titane, zirconium, hafnium et zinc soit seuls, soit mélangés, comme catalyseurs pour la réaction du couplage oxydant du méthane en mode simultané.

Compte-tenu de la présence parfois appréciable de propane et éventuellement d'autres alcanes supérieurs dans le gaz naturel, et compte-tenu éventuellement de la présence d'hydrocarbures légers provenant d'autres unités situées par exemple à proximité de l'unité de couplage oxydant du gaz naturel, il est très avantageux de valoriser, en plus du méthane et de l'éthane, le propane et éventuellement d'autres alcanes supérieurs.

Ainsi, il a été revendiqué (brevet US-A-5 025 108) un procédé d'oxypyrolyse du gaz naturel dans lequel le gaz naturel est séparé en deux fractions, une première fraction riche en méthane, qui est oxydée sélectivement par l'oxygène moléculaire en présence d'une masse de contact (réaction de couplage oxydant du méthane ou C.O.M.), pour former un effluent contenant comme produits principaux des hydrocarbures $C_2$ et de l'eau, cet effluent contenant par ailleurs de faibles quantités de CO, $CO_2$, et $H_2$, et une seconde fraction de gaz enrichie en hydrocarbures $C_2^+$ (c'est-à-dire comprenant au moins 2 atomes de carbone par molécule), qui est mélangée avec ledit effluent quand au moins environ 80% en volume de l'oxygène moléculaire a déjà été consommé dans l'étape d'oxydation du méthane, le mélange résultant étant alors pyrolysé.

Une autre possibilité (brevet US-A-5 113 032) consiste, dans un procédé d'oxypyrolyse, à séparer le gaz naturel en trois fractions, c'est-à-dire une première fraction riche en méthane, une deuxième fraction riche en $C_2$ et une troisième fraction riche en hydrocarbures $C_3^+$, et à pyrolyser séparemment lesdites deuxième et troisième fractions afin d'augmenter le rendement en éthylène, ou à pyrolyser ladite deuxième fraction et à valoriser indépendamment la coupe $C_3^+$ dans tout autre procédé connu de l'homme du métier.

La présente invention concerne donc également un procédé de production d'au moins un alkyl tertiobutyl éther au moins partiellement à partir du gaz naturel dans lequel l'éthylène nécessaire à la production d'isobutène est produit par le procédé d'oxypyrolyse revendiqué dans les brevets US-A-5 025 108 et US-A-5 113 032.

Le procédé d'oxyétherification du gaz naturel selon la présente invention présente entre autres les avantages suivants:

a) La synthèse d'au moins un alkyl tertiobutyl éther est réalisée au moyen d'isobutène et d'alcool(s) qui sont préparés au moins partiellement à partir du gaz naturel.

b) La préparation du gaz de synthèse nécessaire à la synthèse d'au moins un alcool ne nécessite pas l'implantation d'une coûteuse unité de réformage du méthane à la vapeur ou d'oxydation partielle. Une simple unité de préréformage permet de produire l'essentiel du gaz de synthèse, le complément étant fourni par l'unité de C.O.M..

c) L'utilisation des oxydes de carbone (CO, $CO_2$) et de l'hydrogène issus de la réaction de C.O.M., en tant que gaz de synthèse pour produire au moins un alcool, permet de mieux valoriser les sous-produits de cette réaction.

Le procédé d'oxyétherification selon l'invention, permettant de synthétiser au moins un alkyl tertiobutyl éther, de préférence respectivement du méthyl tertiobutyl éther ou de l'éthyl tertiobutyl éther, au moins partiellement à partir du gaz naturel , de préférence purifié, comprend les étapes suivantes :

(a) Le préréformage à la vapeur, dans une zone dite zone de préréformage (26), d'une charge comportant principalement du gaz naturel, de préférence pratiquement exempt de $CO_2$, et de manière encore plus préférée purifié, en présence d'une masse de contact, de façon à convertir partiellement cette fraction en un effluent comprenant principalement de l'hydrogène et des oxydes de carbone (CO, $CO_2$). Ladite charge est de préférence riche en méthane, et elle comprend généralement du méthane, de l'éthane, du propane, d'autres hydrocarbures associés au gaz naturel et éventuellement des gaz non hydrocarbonés. Il est possible de procéder, préalablement au préréformage, a la séparation de ladite charge en une première fraction comprenant principalement du méthane, et de manière préférée étant substantiellement exempte d'éthane, de propane et d'autres hydrocarbures associés au gaz naturel, ladite fraction constituant finalement la charge de la zone de préréformage, et en une seconde fraction comprenant principalement des hydrocarbures comportant au moins deux atomes de carbone par molécule (en particulier comprenant principalement de l'éthane et du propane, et qui peut ou non contenir aussi des gaz non hydrocarbonés), que l'on peut soit valoriser au sein du procédé, par exemple en la mélangeant avec la cinquième fraction décrite ci-après à l'étape (c) et en injectant ledit mélange dans la partie de la zone d'oxypyrolyse dans laquelle on réalise la pyrolyse, soit purger. Cette séparation préalable éventuelle peut être réalisée dans une zone de fractionnement spécifique. Toutefois elle est préférentiellement réalisée dans la zone de séparation décrite à l'étape (c) ci-après, afin de minimiser les investissements.

Le procédé de préréformage a par exemple été décrit par British Gas (Hydrocarbon Processing, p.34, décembre 1990). Tous les hydrocarbures $C_2^+$ présents dans la charge se trouvent décomposés en méthane. Ce dernier, par contre, se met en équilibre de réformage avec l'hydrogène, les oxydes de carbone et la vapeur d'eau.

(b) La majeure partie de l'effluent issu de l'étape de préréformage (a) est mélangée (7) avec la majeure partie du second effluent provenant de la zone de C.O.M. (Couplage Oxydant du Methane) décrite ci-après à l'étape (d), préalablement séchée et comprimée.

(c) Le mélange réalisé à l'étape (b) est ensuite envoyé dans une zone de séparation [(10), (21)]qui permet l'obtention des effluents suivants :

- une première fraction comprenant principalement du dioxyde de carbone,
- une deuxième fraction comprenant principalement du méthane,
- une troisième fraction comprenant principalement de l'éthylène,
- une quatrième fraction contenant principalement de l'hydrogène et du monoxyde de carbone,
- une cinquième fraction comprenant principalement l'éthane et les hydrocarbures $C_3^+$.

(d) La réaction de couplage oxydant du méthane (C.O.M.) est réalisée dans une zone dite zone de C.O.M.(6). Dans le cas où l'on procède à une oxypyrolyse du gaz naturel, la zone d'oxypyrolyse comprend alors deux parties successives : une partie de la zone d'oxypyrolyse dans laquelle on réalise le C.O.M. et une partie de la zone d'oxypyrolyse dans laquelle on réalise la pyrolyse; alors ladite zone de C.O.M. est en fait la partie de la zone d'oxypyrolyse dans laquelle on réalise le C.O.M.. La zone de C.O.M. est alimentée par au moins une fraction de gaz préférentiellement enrichie en méthane, de préférence par au moins une partie de la deuxième fraction décrite à l'étape (c). La zone de C.O.M. fournit un premier effluent comprenant principalement le produit de la réaction, c'est-à-dire de l'éthylène, et un second effluent comprenant principalement les sous-produits de la réaction de C.O.M., c'est-à-dire de l'hydrogène, du monoxyde de carbone et du dioxyde de carbone.

Dans le cas de la présence d'une zone d'oxypyrolyse, il est préférable d'alimenter la partie de la zone d'oxypyrolyse dans laquelle on réalise la pyrolyse par la majeure partie de la cinquième fraction décrite à l'étape (c) comme cela est décrit dans le brevet US-A-5 025 108 cité précédemment. De manière encore plus préférée, ladite cinquième fraction est séparée en un premier flux contenant principalement de l'éthane et en un second flux contenant principalement les hydrocarbures $C_3^+$, de facon soit à alimenter la partie de la zone d'oxypyrolyse dans laquelle on réalise la pyrolyse avec au moins une partie desdits deux flux qui seront pyrolysés séparemment, comme cela est décrit dans le brevet US-A-5 113 032 cité précédemment, soit à alimenter la partie de la zone d'oxypyrolyse dans laquelle on réalise la pyrolyse avec la majeure partie dudit premier flux et à purger la majeure partie dudit second flux.

(e) La majeure partie du premier effluent obtenu à l'étape (d), auquel de préférence on a ajouté la majeure partie de la troisième fraction décrite à l'étape (c), est dimérisée dans une zone dite zone de dimérisation (29), permettant l'obtention d'un effluent comprenant principalement du n-butène.

(f) La majeure partie de l'effluent obtenu à l'étape (e) est isomérisée dans une zone dite zone d'isomérisation (32), permettant l'obtention d'un efflluent comprenant principalement de l'isobutène.

(g) La synthèse d'au moins un alcool, de préférence respectivement du méthanol ou de l'éthanol, est réalisée dans une zone dite zone de synthèse d'alcool(s) (34), à partir d'une partie, de préférence la majeure partie, de la quatrième fraction obtenue à l'étape (c) et d'une partie, de préférence la majeure partie, de la première fraction obtenue à l'étape (c) ainsi que éventuellement d'une partie, de préférence la majeure partie, du second effluent obtenu à l'étape (d), lesdites parties de fractions ou éventuellement de second effluent étant comprimées et de préférence mélangées ensemble en totalité avant l'introduction dans la zone de synthèse d'alcool(s) de façon à former au moins une partie du gaz de synthèse nécessaire à la fabrication d'alcool(s). dans la zone de synthèse d'alcool(s). Une autre partie du gaz de synthèse nécessaire à la fabrication d'alcool(s) peut être éventuellement importée à l'entrée de la zone de synthèse d'alcool(s), en provenance d'une autre unité présente sur le site ou bien en provenance d'un autre site.

Dans le cas particulier de la synthèse du méthanol, qui peut être une synthèse industrielle, une caractéristique du procédé selon la présente invention vient de ce que l'on combine tous les flux alimentant la zone de synthèse d'alcool(s) pour que le gaz alimentant ladite zone ait un rapport $H_2/(2CO + 3CO_2)$ généralement égal approximativement à 1, c'est-à-dire généralement compris entre 0,8 et 1,2, de préférence compris entre 0,9 et 1,1.

Dans le cas particulier de la synthèse de l'éthanol, il est possible de procéder en deux étapes : en première étape on réalise la synthèse de méthanol; puis, dans une seconde étape, on réalise la transformation de la majeure partie du méthanol synthétisé en première étape en éthanol, par exemple par homologation. Il est aussi possible de procéder à la synthèse d'une autre partie de l'éthanol par hydratation de l'éthylène.

D'autre part, il est possible que la zone de synthèse d'alcool(s) soit une zone présente sur le site que l'on utilise partiellement pour l'étape (g) du procédé selon l'invention.

Finalement on obtient un effluent de la zone de synthèse d'alcool(s) comprenant principalement au moins un alcool, de préférence respectivement du méthanol ou de l'éthanol.

(h) La majeure partie de l'effluent obtenu à l'étape (f) est combinée à au moins une partie, de préférence la majeure partie, de l'effluent obtenu à l'étape (g) qui est complété éventuellement par l'importation, en provenance d'une autre unité présente sur le site ou bien en provenance d'un autre site, d'un autre effluent comprenant principalement au moins un alcool, de façon à synthétiser au moins un alkyl tertiobutyl éther, de préférence respectivement du MTBE ou de l'ETBE, dans une zone dite zone de synthèse d'alkyl tertiobutyl éther(s) (37).

En ce qui concerne la masse de contact de zone de préréformage (étape (a) du procédé selon l'invention), on peut utiliser toute masse de contact connue pour cet usage comme par exemple des masses de contact à base de nickel dispersé sur un oxyde présentant de préférence une grande surface spécifique telles que par exemple une alumine ou une alumine stabilisée.

En ce qui concerne la masse de contact utilisée dans la zone réactionnelle réalisant le couplage oxydant du méthane, c'est-à-dire l'oxydation sélective du méthane en hydrocarbures supérieurs (étape (d) du procédé selon l'invention), on préfère utiliser une masse de contact qui vérifie les trois conditions suivantes :

1) elle est capable d'opérer dans les conditions normales du couplage oxydant, par exemple à une température généralement comprise entre 650 et 1200°C, de préférence entre 650 et 950°C,

2) elle produit des hydrocarbures $C_2^+$ avec une sélectivité généralement égale à au moins 65% pour une conversion de 15% du méthane, et 3) elle maintient son activité et sa sélectivité pendant de nombreuses heures de fonctionnement.

Les masses de contact qui correspondent aux conditions ci-dessus et dont l'emploi est donc préféré pour le procédé selon l'invention sont généralement celles qui contiennent des oxydes et/ou des carbonates de métaux alcalins (tels que le lithium, le sodium, le potassium, le rubidium et le césium), de métaux alcalino-terreux (tels que le béryllium, le magnésium, le calcium, le strontium et le baryum) et de métaux des terres rares (tels que l'yttrium, le lanthane, le praséodyme, le néodyme, le samarium, le gadolinium, le terbium, le dysprosium, le holmium, l'erbium, le thulium, l'ytterbium, l'europium et le lutétium), soit seuls (comme dans les cas des terres rares et des alcalino-terreux), soit en mélange (comme dans les cas des métaux alcalino-terreux dopés avec des métaux alcalins et des métaux des terres rares dopés avec des métaux alcalins et /ou alcalino-terreux). D'autres masses de contact qui correspondent aux conditions ci-dessus sont celles qui contiennent des oxydes et/ou des carbonates de titane, zirconium, hafnium, manganèse, cérium, étain, indium, germanium, plomb, antimoine, zinc et bismuth, préférablement avec un ou plusieurs oxydes et/ou carbonates de métaux alcalins, alcalino-terreux, terres rares et silice. Les masses de contact ci-dessus désignées sont efficaces seules ou dopées avec des halogénures, des oxydes de phosphore ou de soufre. Les masses de contact ne sont toutefois pas limitées aux formules indiquées ci-dessus : il est aussi possible plus généralement d'utiliser toute masse de contact prévue pour cet usage.

La fraction de gaz préférentiellement enrichie en méthane et soumise à l'oxydation (étape (d) du procédé selon l'invention) peut être utilisée sans diluant ou peut être diluée par au moins un gaz inerte tels que l'azote, le dioxyde de carbone ou la vapeur d'eau. Pour des raisons de sécurité et pour éviter une élevation de température trop importante qui nuirait à la sélectivité de l'opération, la teneur en oxygène dans le méthane ne peut généralement pas dépasser 40% molaire; elle est donc généralement comprise entre 0,1 et 40% molaire, de préférence entre 5 et 25% molaire.

La température de la réaction de couplage oxydant (C.O.M.) (étape (d) du procédé selon l'invention), est généralement comprise entre 650 et 1200°C, de préférence entre 650 et 950°C.

La pression totale dans la zone de C.O.M. (étape (d) du procédé selon l'invention) est généralement comprise entre 1 et 100 bar (1 bar = $10^5$ Pa), et de préférence entre 1 et 20 bar. Le temps de contact (c'est-à-dire le temps nécessaire pour effectuer la consommation d'au moins 80% de l'oxygène moléculaire dans la charge) est compris habituellement entre $10^{-6}$ et 1 seconde, de préférence entre $10^{-6}$ et $10^{-1}$ seconde.

La réaction de C.O.M. peut être mise en oeuvre dans tout type de réacteur, en particulier dans un réacteur à lit fixe, à lit bouillonnant, à lit circulant ou à lit fluidisé, de préférence dans un réacteur à lit fixe.

En ce qui concerne la masse de contact utilisée pour la réaction de dimérisation de l'éthylène (étape (e) du procédé selon l'invention), il est possible d'utiliser toute masse de contact ou tout catalyseur soluble connu pour cet usage, comme par exemple un sel de nickel déposé sur un support oxyde tel que l'alumine ou, par exemple, un mélange de complexes solubles comprenant du nickel et un acide de Lewis tel que le mélange d'un carboxylate de nickel et d'un trialkylaluminium. On peut également utiliser le mélange d'un titanate d'alkyl et d'un alkyl ou hydrocarbyl aluminium permettant de dimériser sélectivement l'éthylène en butène-1 ainsi que l'indiquent les brevets US-A-4 532 370, US-A-4 615 998 et US-A-4 721 762.

En ce qui concerne la masse de contact utilisée pour la réaction d'isomérisation du n-butène en isobutène (étape (f) du procédé selon l'invention), on peut utiliser toute masse de contact connue pour cet usage, et plus particulièrement toute masse de contact présentant une acidité sensiblement faible telles que les alumines éventuellement dopées, les silice-alumines, les silice-zircones ou les zéolithes. Il est en particulier possible et avantageux d'utiliser les masses de contact décrites dans la demande de brevet français 92/10.949.

La synthèse d'au moins un alcool (étape (g) du procédé selon l'invention) se fait selon tout procédé connu de l'homme du métier. Ainsi, la synthèse du méthanol à partir du gaz de synthèse est un procédé industriel, généralement opéré en présence de catalyseurs à base de cuivre et précedemment décrit par exemple dans les brevets US-A-3 388 972 et US-A-3 546 140 de la société C.C.I. et US-A-3 923 694 de la société I.C.I.. On peut utiliser dans ce procédé toute masse de contact connue pour cet usage, tels par exemple les catalyseurs à base de cuivre aluminium et zinc décrits dans le brevet US-A-5 112 591. La synthèse de mélanges d'alcools a par ailleurs été décrite par la demanderesse, par exemple dans les brevets US-A-4 780 481 et US-A-4 791 141. On peut utiliser dans ce procédé toute masse de contact connue pour cet usage. Des catalyseurs à base de métaux du groupe VIII tels que par exemple le nickel, le fer et le molybdène, ont été décrits dans la littérature, on peut par exemple utiliser les catalyseurs à base de cuivre et de cobalt décrits dans le brevet US-A-4 791 141.

En ce qui concerne la masse de contact utilisée pour la réaction de synthèse d'au moins un alkyl tertiobutyl éther (étape (h) du procédé selon l'invention), on peut utiliser toute masse de contact connue pour cet usage, tels que par exemple les résines acides comme par exemple les résines sulfoniques ou tout oxyde acide ou zéolithe présentant une acidité adéquate. Une telle synthèse est par exemple décrite dans les brevets US-A-4 847 431, US-A-5 013 407 et US-A-4 847 430.

Il est également possible d'utiliser le procédé selon l'invention pour préparer simultanément au moins un alkyl tertiobutyl éther ainsi que du carburant liquide (par exemple de l'essence et/ou du gasoil) par dimérisation et/ou oligomérisation d'une partie des oléfines produites lors de l'étape de couplage oxydant du méthane (étape (d) du procédé selon l'invention), comme cela a été décrit par exemple dans les brevets US-A-5 025 108 et US-A-5 113 032. Dans ce cas, il est possible de supprimer l'étape (a) de préréformage du procédé selon l'invention et seul le gaz de synthèse issu de l'étape (d) (c'est-à-dire le second effluent de la zone de C.O.M.) est alors utilisé pour la production d'alcool(s). Ce (ou ces) alcool(s) est(sont) ensuite avantageusement transformé(s) en alkyl tertiobutyl éther(s) en le(s) faisant réagir, dans l'étape (h) du procédé selon l'invention, avec l'isobutène produit par dimérisation et isomérisation (étapes (e) et (f) du procédé selon l'invention) de la fraction d'éthylène résiduelle qui n'a pas été transformée en carburant liquide. Il est ainsi possible d'obtenir, au moyen du procédé selon l'invention, une base carburant liquide très performante, telle que de l'essence à haut indice d'octane, en mélangeant le carburant produit avec au moins une partie de l'(des) alkyl tertiobutyl éther(s) obtenu(s) simultanément.

Finalement, l'invention concerne aussi un procédé de production simultanée d'au moins un alkyl tertiobutyl éther et de carburant liquide à partir de gaz naturel, ledit procédé comprenant les étapes suivantes :

(1) la majeure partie du second effluent provenant de la zone de COM décrit ci-après à l'étape (2) préalablement séchée et comprimée est envoyée dans une zone de séparation qui permet l'obtention des effluents suivants :

- une première fraction comprenant principalement du dioxyde de carbone
- une deuxième fraction comprenant principalement du méthane
- une troisième fraction comprenant principalement de l'éthylène
- une quatrième fraction comprenant principalement de l'hydrogène et du monoxyde de carbone,
- une cinquième fraction comprenant principalement l'éthane et les hydrocarbures C3+

(2) la réaction de couplage oxydant du méthane est réalisée dans une zone de COM, permettant l'obtention d'un premier effluent comprenant principalement de l'éthylène et d'un second effluent comprenant principalement de l'hydrogène, du monoxyde de carbone et du dioxyde de carbone.

(3) une partie du premier effluent obtenu à l'étape (2) est dimérisée dans une zone de dimérisation permettant l'obtention d'un effluent comprenant principalement du n-butène.

(4) la majeure partie de l'effluent obtenu à l'étape (3) est isomérisée dans une zone d'isomérisation permettant l'obtention d'un effluent comprenant principalement de l'isobutène.

(5) La synthèse d'au moins un alcool est réalisée dans une zone de synthèse d'alcool(s), à partir d'une partie de la quatrième fraction obtenue à l'étape (1) et d'une partie de la première fraction obtenue à l'étape (1), lesdites parties étant comprimées avant l'introduction dans la zone de synthèse d'alcool(s), permettant l'obtention d'un effluent comprenant principalement au moins un alcool.

(6) une autre partie du premier effluent obtenu à l'étape (2) ci après subit au moins une des réactions suivantes : dimérisation, oligomérisation dans une zone réactionnelle, permettant l'obtention d'un effluent comprenant principalement du carburant liquide.

(7) La majeure partie de l'effluent obtenu à l'étape (4) est combinée à au moins une partie de l'effluent obtenu à l'étape (5), de façon à synthétiser au moins un alkyl tertiobutyl éther, dans une zone de synthèse d'alkyl tertiobutyl éther(s).

(8) la majeure partie de l'effluent obtenu à l'étape (6) est mélangée avec une partie de l' (des) alkyl tertiobutyl éther(s), permettant l'obtention d'une base carburant liquide.

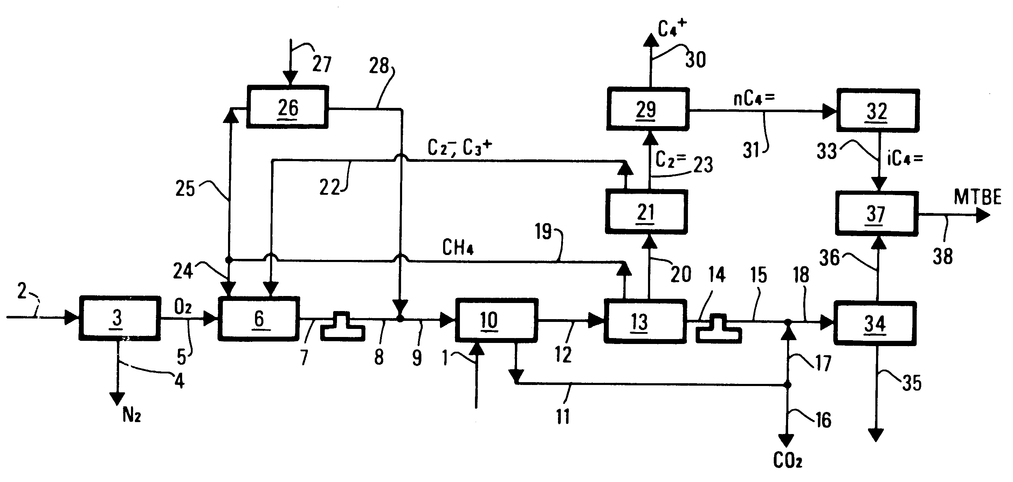

L'exemple suivant non limitatif représente un mode de réalisation du procédé selon l'invention, qui peut être employé pour effectuer l'oxyétherification du gaz naturel selon l'invention, et qui est illustré par la Figure.

EXEMPLE (selon l'invention)

On se propose de produire 100 000 T/an de MTBE. A cet effet on part avec 860 kmoles/h d'un gaz naturel de composition suivante (en mole %) :

| | |
|---|---|
| $CH_4$ : | 88,7 |
| $C_2$ : | 5,6 |
| $C_3+$ : | 2,6 |
| $CO_2$ : | 2,3 |
| $H_2S$ : | 0,8 |
| | $\overline{100,00}$ |

Après épuration et récupération des GPL, on envoie dans la conduite (1) 810 kmoles/h d'un gaz ne contenant pratiquement que des hydrocarbures comportant un ou deux atome(s) de carbone par molécule que l'on décarbonate dans l'unité (10).

Dans l'unité (13) ci-après désignée sous le vocable de "boîte froide", le produit arrivant par la conduite (12) de l'unité (10) est séparé en trois fractions.

La première fraction, évacuée par la conduite (14) puis après compression par la conduite (15), est formée éventuellement d'incondensables, d'hydrogène et de monoxyde de carbone. Cette fraction va servir de gaz d'alimentation à l'unité de synthèse du méthanol (34). La composition de cette fraction ne correspond pas à la stoechiométrie de la synthèse du méthanol. Alors on s'arrange pour prélever par la conduite (17), dans la purge soutirée par la conduite (11) de l'unité de décarbonatation (10), suffisamment de $CO_2$ pour que, quand on ajoute les gaz des conduites (15) et (17), on obtienne un gaz alimentant l'unité (34) par la conduite (18) qui a un rapport $H_2/(2CO + 3CO_2)$ approximativement égal à 1. Ce qui provient de la purge soutirée de l'unité (10) et qui n'est pas prélevé par la conduite (17) est évacué par la conduite (16). Ainsi, sur les 187,5 kmoles/h de purge provenant par la conduite (11) de l'unité (10), on en prélève 107,5 par la conduite (17) et on en rejette 80 kmoles/h à l'atmosphère par la conduite (16).

Une partie (5254 kmoles/h) de la deuxième fraction contenant essentiellement du méthane et issue de l'unité (13) par la conduite (19), est envoyée dans l'unité (6) de couplage oxydant par la conduite (24) pour subir la réaction d'oxypyrolyse. L'autre partie de ladite deuxième fraction (764,5 kmoles/h) est envoyée par la conduite (25) sur l'unité de préréformage (26), afin de produire l'hydrogène nécessaire à la stoechiométrie de la synthèse du méthanol.

La troisième fraction, correspondant à 484,5 kmoles/h de $C_2^+$, est envoyée par la conduite (20) sur l'unité de fractionnement (21).

Dans l'unité (26), les 764,5 kmoles/h de gaz sont transformées grâce à de la vapeur injectée par la conduite (27)

7

en 1038,5 kmoles/h de gaz (compté sec) de composition suivante :

| | |
|---|---|
| CO : | 0,51 |
| $CO_2$ : | 6,12 |
| $H_2$ : | 25,86 |
| $C_1$: | 67,51 |
| | $\overline{100,00}$ |

Ce gaz arrivant par la conduite (28) est mélangé avec le gaz effluent de l'unité (6) par la conduite (7) puis après séchage et compression par la conduite (8), et le mélange arrivant par la conduite (9) est décarbonaté dans l'unité (10). Cette décarbonatation est nécessaire pour éviter la formation de carboglace dans la boîte froide (13).

L'unité (21) sépare d'abord les $C_3^+$ que l'on renvoie vers l'unité (6) par la conduite (22) au niveau de la section de pyrolyse installée en aval du réacteur C.O.M.. La fraction restante de la conduite (20) initiale est distillée et donne de l'éthylène soit 296,5 kmoles/h ainsi que de l'éthane soit 172 kmoles/h. L'éthane est également envoyé sur l'unité (6) par la conduite (22) pour être pyrolysé en éthylène, comme cela est décrit dans le brevet US-A-5 025 108. Cette unité (6) est alimentée en oxygène par la conduite (5), ledit oxygène provenant d'une unité de distillation de l'air (3) alimentée en air par la conduite (2) et produisant de l'azote par la conduite (4). Enfin, l'éthylène issu de l'unité (21) soit 296,5 kmoles/h est envoyé par la conduite (23) dans l'unité de dimérisation (29). On obtient ainsi 142 kmoles/h de n-butène en même temps qu'une fraction lourde $C_4^+$ qu'on purge par la conduite (30) soit 620 kg/h.

Le butène linéaire est envoyé par la conduite (31) sur l'unité (32) qui est une unité d'isomérisation du n-butène en isobutène. Cet isobutène est envoyé par la conduite (33) sur l'unité (37) de synthèse du méthyl tertiobutyl éther (MTBE) qui reçoit par ailleurs du méthanol provenant de l'unité de synthèse du méthanol (34) par la conduite (36).

On obtient finalement dans la conduite (38) 12487 kg/h d'éther soit annuellement environ 100 000 T/an. La purge est évacuée par la conduite (35). Dans le cas présent, tout le méthanol nécessaire à la fabrication du MTBE, soit 4650 kg/an, a pu être fabriqué à partir du gaz effluent de l'unité (6) d'oxypyrolyse.

**Revendications**

1. Procédé de production d'au moins un alkyl tertiobutyl éther au moins partiellement à partir du gaz naturel, ledit procédé comprenant les étapes suivantes :

   (a) Le préréformage à la vapeur, dans une zone de préréformage (26) d'une charge comprenant principalement du gaz naturel, en présence d'une masse de contact, de façon à obtenir un effluent comprenant principalement de l'hydrogène, du monoxyde de carbone et du dioxyde de carbone.

   (b) La majeure partie de l'effluent issu de l'étape de préréformage (a) est mélangée (7) avec la majeure partie du second effluent provenant de la zone de C.O.M. (Couplage Oxydant du Méthane) décrit ci-après à l'étape (d) préalablement séché et comprimé.

   (c) Le mélange réalisé à l'étape (b) est ensuite envoyé dans une zone de séparation [(10), (21)] qui permet l'obtention des effluents suivants :

   - une première fraction comprenant principalement du dioxyde de carbone,
   - une deuxième fraction comprenant principalement du méthane,
   - une troisième fraction comprenant principalement de l'éthylène,
   - une quatrième fraction contenant principalement de l'hydrogène et du monoxyde de carbone,
   - une cinquième fraction comprenant l'éthane et les hydrocarbures $C_3^+$.

   (d) La réaction de couplage oxydant du méthane est réalisée dans une zone (6) de C.O.M., permettant l'obtention d'un premier effluent (23) comprenant principalement de l'éthylène, et d'un second effluent comprenant principalement de l'hydrogène, du monoxyde de carbone et du dioxyde de carbone.

   (e) La majeure partie du premier effluent obtenu à l'étape (d) est dimérisée dans une zone de dimérisation (29), permettant l'obtention d'un effluent comprenant principalement du n-butène.

(f) La majeure partie de l'effluent obtenu à l'étape (e) est isomérisée dans une zone d'isomérisation (32), permettant l'obtention d'un efflluent comprenant principalement de l'isobutène.

(g) La synthèse d'au moins un alcool est réalisée dans une zone de synthèse d'alcool(s) (34), à partir d'une partie de la quatrième fraction obtenue à l'étape (c) et d'une partie de la première fraction obtenue à l'étape (c), lesdites parties étant comprimées avant l'introduction dans la zone de synthèse d'alcool(s), permettant l'obtention d'un effluent comprenant principalement au moins un alcool.

(h) La majeure partie de l'effluent obtenu à l'étape (f) est combinée à au moins une partie de l'effluent obtenu à l'étape (g), de façon à synthétiser au moins un alkyl tertiobutyl éther, dans une zone de synthèse d'alkyl tertiobutyl éther(s) (37).

2. Procédé selon la revendication 1 dans lequel on procède, préalablement au préréformage, à la séparation de ladite charge en une première fraction comprenant principalement du méthane qui constitue finalement la charge réelle pour l'étape (a) et en une seconde fraction comprenant principalement des hydrocarbures comportant au moins deux atomes de carbone par molécule.

3. Procédé selon la revendication 2 dans lequel ladite séparation est effectuée dans la zone de séparation décrite à l'étape (c).

4. Procédé selon l'une des revendications 1 à 3 dans lequel on alimente la zone de C.O.M. par au moins une partie de la deuxième fraction décrite à l'étape (c).

5. Procédé selon l'une des revendications 1 à 4 dans lequel la zone de C.O.M. est la partie de la zone d'oxypyrolyse dans laquelle on réalise le C.O.M..

6. Procédé selon la revendication 5 dans lequel on alimente la partie de la zone d'oxypyrolyse dans laquelle on réalise la pyrolyse par la cinquième fraction décrite à l'étape (c).

7. Procédé selon l'une des revendications 1 à 6 dans lequel on introduit en outre dans la zone de synthèse d'alcool(s) une partie comprimée du second effluent obtenu à l'étape (d).

8. Utilisation du procédé selon l'une des revendications 1 à 7 pour la synthèse de méthyl tertiobutyl éther.

9. Utilisation du procédé selon l'une des revendications 1 à 7 pour la synthèse d'éthyl tertiobutyl éther.

10. Procédé de production simultanée d'au moins un alkyl tertiobutyl éther et de carburant liquide à partir de gaz naturel, ledit procédé comprenant les étapes suivantes :

(1) la majeure partie du second effluent provenant de la zone de COM décrit ci-après à l'étape (2) préalablement séchée et comprimée est envoyée dans une zone de séparation qui permet l'obtention des effluents suivants :

- une première fraction comprenant principalement du dioxyde de carbone
- une deuxième fraction comprenant principalement du méthane
- une troisième fraction comprenant principalement de l'éthylène
- une quatrième fraction comprenant principalement de l'hydrogène et du monoxyde de carbone,
- une cinquième fraction comprenant principalement l'éthane et les hydrocarbures $C_3{}^+$.

(2) la réaction de couplage oxydant du méthane est réalisée dans une zone de COM, permettant l'obtention d'un premier effluent comprenant principalement de l'éthylène et d'un second effluent comprenant principalement de l'hydrogène, du monoxyde de carbone et du dioxyde de carbone.
(3) une partie du premier effluent obtenu à l'étape (2) est dimérisée dans une zone de dimérisation permettant l'obtention d'un effluent comprenant principalement du n-butène.
(4) la majeure partie de l'effluent obtenu à l'étape (3) est isomérisée dans une zone d'isomérisation permettant l'obtention d'un effluent comprenant principalement de l'isobutène.
(5) La synthèse d'au moins un alcool est réalisée dans une zone de synthèse d'alcool(s), à partir d'une partie de la quatrième fraction obtenue à l'étape (1) et d'une partie de la première fraction obtenue à l'étape (1), lesdites parties étant comprimées avant l'introduction dans la zone de synthèse d'alcool(s), permettant l'obtention d'un effluent comprenant principalement au moins un alcool.

(6) une autre partie du premier effluent obtenu à l'étape (2) ci après subit au moins une des réactions suivantes : dimérisation, oligomérisation dans une zone réactionnelle, permettant l'obtention d'un effluent comprenant principalement du carburant liquide.

(7) La majeure partie de l'effluent obtenu à l'étape (4) est combinée à au moins une partie de l'effluent obtenu à l'étape (5), de façon à synthétiser au moins un alkyl tertiobutyl éther, dans une zone de synthèse d'alkyl tertiobutyl éther(s).

(8) la majeure partie de l'effluent obtenu à l'étape (6) est mélangée avec une partie de l' (des) alkyl tertiobutyl éther(s), permettant l'obtention d'une base carburant liquide.

**Claims**

1. A process for the production of at least one alkyl tertiobutyl ether at least partially from natural gas, said process comprising the following steps:

    (a) steam prereforming a feedstock containing mainly natural gas in a prereforming zone in the presence of a contact mass to produce an effluent containing mainly hydrogen, carbon monoxide and carbon dioxide,
    (b) mixing a major portion of the effluent from prereforming step (a) (7) with a major portion of the second effluent from the OCM zone described in step (d) below, which has been dried and compressed,
    (c) sending the mixture formed in step (b) to a separation zone [(10) to (21)] to produce the following effluents:

    - a first fraction containing mainly carbon dioxide,
    - a second fraction containing mainly methane,
    - a third fraction containing mainly ethylene,
    - a fourth fraction containing mainly hydrogen and carbon monoxide,
    - a fifth fraction containing mainly ethane and $C_3^+$ hydrocarbons,

    (d) carrying out oxidative coupling of methane(OCM) (6) in an OCM zone to produce a first effluent (23) containing mainly ethylene, and a second effluent containing mainly hydrogen, carbon monoxide and carbon dioxide,
    (e) dimerising a major portion of the first effluent obtained from step (d) in a dimerisation zone (29) to produce an effluent containing mainly n-butene,
    (f) isomerising a major portion of the effluent obtained from step (e) in an isomerisation zone (32) to produce an effluent containing mainly isobutene,
    (g) synthesizing at least one alcohol in an alcohol synthesis zone (34), from a portion of the fourth fraction obtained from step (c) and a portion of the first fraction obtained from step (c), said portions being compressed before their introduction into the alcohol synthesis zone to form an effluent containing at least one alcohol,
    (h) combining a major portion of the effluent obtained in step (f) with at least a portion of the effluent obtained in step (g) to synthesize at least one alkyl tertiobutyl ether in an alkyl tertiobutyl ether synthesis zone (37).

2. Process according to claim 1 wherein, prior to prereforming, said feedstock is separated into a first fraction containing mainly methane which constitutes the actual feedstock for step (a), and a second fraction containing mainly hydrocarbons containing at least two carbon atoms per molecule.

3. Process according to claim 2, wherein said separation is carried out in the separation zone described in step (c).

4. Process according to any one of claims 1 to 3 wherein the supply to the OCM zone is constituted by at least a portion of the second fraction described in step (c).

5. Process according to any one of claims 1 to 4 wherein the OCM zone is constituted by the section of the oxypyrolysis zone in which the OCM is carried out.

6. Process according to claim 5 wherein the section of the oxypyrolysis zone in which pyrolysis is carried out is supplied by the fifth fraction described in step (c).

7. Process according to any one of claims 1 to 6 wherein a compressed portion of the second effluent obtained from step (d) is introduced into the alcohol synthesis zone.

8. Use of a process according to any one of claims 1 to 7 for the synthesis of methyl tertiobutyl ether.

9. Use of a process according to any one of claims 1 to 7 for the synthesis of ethyl tertiobutyl ether.

10. A process for the simultaneous production of at least one alkyl tertiobutyl ether and a liquid fuel from natural gas, said process comprising the following steps:

(1) a major portion of the second effluent from the OCM zone described in step (2) is dried and compressed then sent to a separation zone to produce the following effluents:

- a first fraction containing mainly carbon dioxide,
- a second fraction containing mainly methane,
- a third fraction containing mainly ethylene,
- a fourth fraction containing mainly hydrogen and carbon monoxide,
- a fifth fraction containing mainly ethane and $C_3^+$ hydrocarbons,

(2) oxidative coupling of methane is carried out in an OCM zone to produce a first effluent containing mainly ethylene and a second effluent containing mainly hydrogen, carbon monoxide and carbon dioxide,
(3) a portion of the first effluent obtained from step (2) is dimerised in a dimerisation zone to produce an effluent containing mainly n-butene,
(4) a major portion of the effluent obtained from step (3) is isomerised in an isomerisation zone to produce an effluent containing mainly isobutene,
(5) synthesis of at least one alcohol is carried out in an alcohol synthesis zone using a portion of the fourth fraction obtained from step (1) and a portion of the first fraction obtained from step (1), said portions being compressed before introduction into the alcohol synthesis zone to form an effluent containing mainly at least one alcohol,
(6) a further portion of the first effluent obtained from step (2) undergoes at least one of the following reactions: dimerisation or oligomerisation in a reaction zone, to produce an effluent containing mainly liquid fuel,
(7) a major portion of the effluent obtained from step (4) is combined with at least a portion of the effluent obtained from step (5) to synthesize at least one alkyl tertiobutyl ether in an alkyl tertiobutyl ether synthesis zone,
(8) a major portion of the effluent obtained from step (6) is mixed with a portion of the alkyl tertiobutyl ether(s) to produce a liquid fuel base.

**Patentansprüche**

1. Verfahren zur Herstellung mindestens eines Alkyl-tert-butyl-ethers mindestens zum Teil aus Erdgas, das die folgenden Stufen umfaßt:

(a) Dampf-Vorreformierung einer hauptsächlich Erdgas enthaltenden Beschickung in einer Vorreformierungszone (26) in Gegenwart einer Kontaktmasse (Katalysatormasse) zur Herstellung eines Abstroms, der hauptsächlich Wasserstoff, Kohlenmonoxid und Kohlendioxid enthält;

(b) Mischen (7) des Hauptteils des aus der Vorreformierungsstufe (a) stammenden Abstroms mit dem Hauptteil des zweiten Abstroms, der aus der C.O.M.-Zone (oxidativen Methan-Kupplungszone), wie sie nachstehend in der Stufe (d) beschrieben wird, stammt, der vorher getrocknet und komprimiert worden ist;

(c) anschließendes Einführen der in der Stufe (b) erhaltenen Mischung in eine Trennzone [(10), (21)], in der die folgenden Abströme erhalten werden:

- eine erste Fraktion, die hauptsächlich Kohlendioxid enthält,
- eine zweite Fraktion, die hauptsächlich Methan enthält,
- eine dritte Fraktion, die hauptsächlich Ethylen enthält,
- eine vierte Fraktion, die hauptsächlich Wasserstoff und Kohlenmonoxid enthält,
- eine fünfte Fraktion, die Ethan und die $C_{3+}$-Kohlenwasserstoffe enthält;

(d) Durchführung der oxidativen Methan-Kupplungsreaktion in einer C.O.M.-Zone (6), wobei man erhält einen ersten Abstrom (23), der hauptsächlich Ethylen enthält, und einen zweiten Abstrom, der hauptsächlich Wasserstoff, Kohlenmonoxid und Kohlendioxid enthält;

(e) Dimerisieren des Hauptteils des in der Stufe (d) erhaltenen ersten Abstroms in einer Dimerisierungszone (29), wobei man einen Abstrom erhält, der hauptsächlich n-Buten enthält;

(f) Isomerisieren des Hauptteils des in der Stufe (e) erhaltenen Abstroms in einer Isomerisierungszone (32), wobei man einen Abstrom erhält, der hauptsächlich Isobuten enthält;

(g) Synthetisieren mindestens eines Alkohols in einer Alkohol-Synthese-Zone (34), wobei man von einem Teil der in der Stufe (c) erhaltenen vierten Fraktion und einem Teil der in der Stufe (c) erhaltenen ersten Fraktion ausgeht, die beide vor der Einführung in die Alkohol-Synthese-Zone komprimiert werden, wobei man einen Abstrom erhält, der hauptsächlich mindestens einen Alkohol enthält; und

(h) Kombinieren des Hauptteils des in der Stufe (f) erhaltenen Abstroms mit mindestens einem Teil des in der Stufe (g) erhaltenen Abstroms zum Synthetisieren mindestens eines Alkyl-tert-butyl-ethers in einer Alkyl-tert-butyl-ether-Synthesezone (37).

2.  Verfahren nach Anspruch 1, bei dem man vor der Vorreformierung die genannte Beschickung auftrennt in eine erste Fraktion, die hauptsächlich Methan enthält, die schließlich die eigentliche Beschickung für die Stufe (a) darstellt, und in eine zweite Fraktion, die hauptsächlich Kohlenwasserstoffe mit mindestens zwei Kohlenstoffatomen pro Molekül enthält.

3.  Verfahren nach Anspruch 2, bei dem die genannte Auftrennung in der in der Stufe (c) beschriebenen Trennzone durchgeführt wird.

4.  Verfahren nach einem der Ansprüche 1 bis 3, bei dem man der C.O.M.-Zone mindestens einen Teil der in der Stufe (c) beschriebenen zweiten Fraktion zuführt.

5.  Verfahren nach einem der Ansprüche 1 bis 4, bei dem die C.O.M.-Zone der Teil der Oxypyrolyse-Zone ist, in der man die oxidative Kupplung von Methan (C.O.M.) durchführt.

6.  Verfahren nach Anspruch 5, bei dem man in den Teil der Oxypyrolyse-Zone, in dem die Pyrolyse durchgeführt wird, die in der Stufe (c) beschriebene fünfte Fraktion einführt.

7.  Verfahren nach einem der Ansprüche 1 bis 6, bei dem man außerdem in die Alkohol-Synthese-Zone einen komprimierten Teil des in der Stufe (d) erhaltenen zweiten Abstrom einführt.

8.  Anwendung des Verfahrens nach einem der Ansprüche 1 bis 7 zur Synthese von Methyl-tert-butyl-ether.

9.  Anwendung des Verfahrens nach einem der Ansprüche 1 bis 7 zur Synthese von Ethyl-tert-butyl-ether.

10. Verfahren zur gleichzeitigen Herstellung mindestens eines Alkyl-tert-butyl-ethers und von flüssigem Treibstoff (Kraftstoff) aus Erdgas, das die folgenden Stufen umfaßt:

(1) Einführen des Hauptteils des zweiten Abstroms, der aus der in der nachstehenden Stufe (2) beschriebenen COM-Zone stammt, der vorher getrocknet und komprimiert worden ist, in eine Trennzone, in der man die folgenden Abströme erhält:

-   eine erste Fraktion, die hauptsächlich Kohlendioxid enthält,
-   eine zweite Fraktion, die hauptsächlich Methan enthält,
-   eine dritte Fraktion, die hauptsächlich Ethylen enthält,
-   eine vierte Fraktion, die hauptsächlich Wasserstoff und Kohlenmonoxid enthält,
-   eine fünfte Fraktion, die hauptsächlich Ethan und die $C_{3+}$-Kohlenwasserstoffe enthält;

(2) Durchführung der oxidativen Methan-Kupplungsreaktion in einer C.O.M.-Zone, wobei man erhält einen ersten Abstrom, der hauptsächlich Ethylen enthält, und einen zweiten Abstrom, der hauptsächlich Wasserstoff, Kohlenmonoxid und Kohlendioxid enthält;

(3) Dimerisieren eines Teils des in der Stufe (2) erhaltenen ersten Abstroms in einer Dimerisierungszone, wobei man einen Abstrom erhält, der hauptsächlich n-Buten enthält;

(4) Isomerisieren des Hauptteils des in der Stufe (3) erhaltenen Abstroms in einer Isomerisierungszone, wobei man einen Abstrom erhält, der hauptsächlich Isobuten enthält;

(5) Synthetisieren mindestens eines Alkohols in einer Alkohol-Synthese-Zone, wobei man von einem Teil der in der Stufe (1) erhaltenen vierten Fraktion und einem Teil der in der Stufe (1) erhaltenen ersten Fraktion ausgeht, die beide vor der Einführung in die Alkohol-Synthese-Zone komprimiert werden, wobei man einen Abstrom erhält, der hauptsächlich mindestens einen Alkohol enthält;

(6) Durchführung mindestens einer der folgenden Reaktionen mit einem anderen Teil des in der Stufe (2) erhaltenen ersten Abstroms: Dimerisierung und Oligomerisierung in einer Reaktionszone, wobei man einen Abstrom erhält, der hauptsächlich flüssigen Treibstoff (Kraftstoff) enthält;

(7) Kombinieren des Hauptteils des in der Stufe (4) erhaltenen Abstroms mit mindestens einem Teil des in der Stufe (5) erhaltenen Abstroms zum Synthetisieren mindestens eines Alkyl-tert-butyl-ethers in einer Alkyl-tert-butyl-ether-Synthesezone; und

(8) Mischen des Hauptteils des in der Stufe (6) erhaltenen Abstroms mit einem Teil des (der) Alkyl-tert-butyl-ether(s), wobei man eine flüssige Treibstoff(Kraftstoff)-Grundlage(-Basis) erhält.